(19) Europäisches Patentamt
European Patent Office
Office européen des brevets

(11) Publication number: 0 015 140
B1

(12) EUROPEAN PATENT SPECIFICATION

(45) Date of publication of patent specification: 14.12.83

(51) Int. Cl.³: C 07 D 261/08 //A01N43/80

(21) Application number: 80300481.1

(22) Date of filing: 20.02.80

# (54) Thermolysis of 2-(3-aryl-isoxazol-5-yl)benzoic acids, salts and halides from 3'-(aryl)-spiro(isobenzofuran-1(3H),5'(4'H)isoxazol)-3-ones.

(30) Priority: 22.02.79 US 13853
09.03.79 US 19049
17.05.79 US 39935

(43) Date of publication of application:
03.09.80 Bulletin 80/18

(45) Publication of the grant of the patent:
14.12.83 Bulletin 83/50

(84) Designated Contracting States:
AT BE CH DE FR GB IT NL SE

(56) References cited:
FR - A - 2 390 441
US - A - 4 032 644

Katritzky, Adv. Heterocyclic Chem., 25(1979) 163-165

(73) Proprietor: MONSANTO COMPANY
Patent Department 800 North Lindbergh Boulevard
St. Louis, Missouri 63166 (US)

(72) Inventor: Liu, Kou Chang
84-06 98th Street
Woodhaven New York New York 11421 (US)
Inventor: Howe, Robert Kenneth
12176 Dunsinane Court
Bridgeton Missouri 63044 (US)

(74) Representative: Nash, Brian Walter et al,
Monsanto Europe S.A. Avenue de Tervuren 270-272 Letter Box No 1
B-1150 Brussels (BE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

Preparation of 2-(3-aryl-isoxazol-5-yl)benzoic acids, salts and halides from 3′-(aryl)-spiro(isobenzofuran-1(3H),5′(4′H)isoxazol)-3-ones

This invention relates to the preparation of 2-(3-Aryl-5-isoxazolyl)benzoic acids, salts or halides thereof by treatment of 3′-(Aryl)-spiro[isobenzofuran-1(3H),5′(4′H)-isoxazol]-3-one with heat, strong base or a reactive halide in water.

Belgian Patent 837,454 discloses such isoxazolyl benzoates to be effective plant growth regulants. Isoxazol-5-yl benzoates are usually prepared by conversion of isoxazolin-5-yl benzoate with N-bromosuccinimide or dichlorodicyanobenzoquinone. However, isoxazolin-5-yl benzoates are prepared from vinyl benzoates which are difficult to prepare.

United States Patent 4032644 describes the preparation of 2-(3-aryl-isoxazol-5-yl) benzoic acids by acid-catalyzed dehydration of 2-(3-aryl-5-hydroxy-2-isoxazolin-5-yl)-benzoic acids. The time of reaction is from 12 to 30 hours. The starting material and the reaction product are both benzoic acids.

In accordance with one aspect of the present invention 2-(3-Aryl-5-isoxazolyl)-benzoic acids having the formula

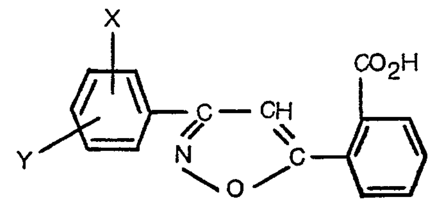

wherein X and Y are independently selected from the group consisting of hydrogen, halogen, $C_{1-5}$ alkyl, $C_{1-5}$ alkoxy, halo-$C_{1-5}$ alkyl, phenoxyphenyl and cyano, are prepared by heating a molten 3′-(Aryl)spiro[isobenzofuran-1(3H),5′(4′H)-isoxazol]-3-one having the formula

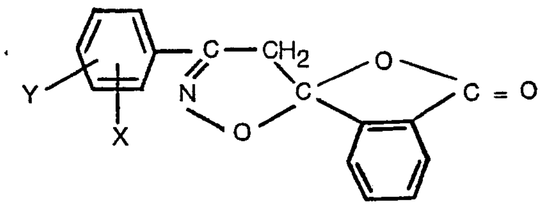

or by heating a solution of a 3′-(Aryl)spiro[isobenzofuran-1(3H),5′(4′H)-isoxazol]-3-one having the said formula in an inert solvent at a temperature ranging from 100°C to the reflux temperature of the solution.

Another aspect of the invention is a process for preparing a salt of a 2-(3-Aryl-5-isoxazolyl)-benzoic acid which comprises heating a spiro compound having the said formula with a base having a $pK_A$ of 11 or more.

3-(Aryl)-spiro[isobenzofuran-1(3H),5′(4′H)-isoxasol]-3-one starting materials are prepared by reaction of a nitrile oxide with 3-methylenephthalide in accordance with the following equation:

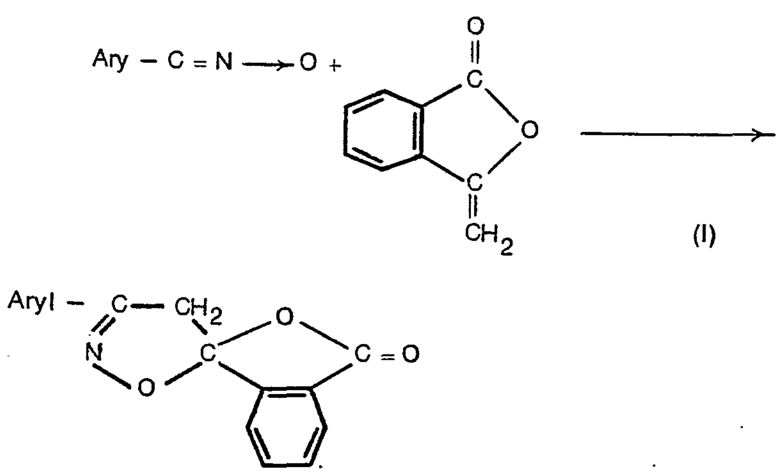

(I)

Aryl is a radical of the following formula

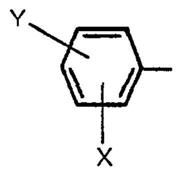

wherein X and Y are independently selected from the group consisting of hydrogen, halogen, $C_{1-5}$ alkyl, $C_{1-5}$ alkoxy, halo-$C_{1-5}$ alkyl, phenoxy, phenyl and cyano. Both straight as well as branched chain alkyl groups are contemplated.

The term "halo-$C_{1-5}$ alkyl" as used herein is understood to mean those $C_{1-5}$ alkyl groups in which at least one, and perhaps all, of the hydrogen atoms have been replaced by halogen atoms. It is to be clearly understood that trifluoromethyl is contemplated as being a halo-$C_{1-5}$ alkyl moiety.

The term "halogen" as used herein includes chlorine, bromine, fluorine and iodine.

In accordance with the first aforesaid aspect of the process of the invention, the benzoic acid is prepared by heating the spiro compound to above its melting point. The conversion is complete in a short period of time, normally less than one hour. Heating is usually carried out at atmospheric pressure at a temperature just above the melting point.

In industrial applications, it may be found to be convenient to dissolve the spiro compound in a suitable solvent and heat the solution to convert the spiro compound to the desired acid. The amount of heat applied is not critical but should be that amount which is sufficient to convert said spiro compound to the desired acid. Conversion at temperatures above 100°C, and especially 125°C up to said reflux temperature, will result in satisfactory conversion. However, heating the solution from temperatures just above the melting point of the spiro starting material to the reflux temperature of the solution is preferred to effect rapid and effective conversion.

The solvent utilized is not critical. Any solvent which is inert to the spiro compound and the desired acid and has a sufficiently high boiling point will suffice. Examples of such suitable solvents are *o*-dichlorobenzene, dodecane, 1,2,3-trimethylbenzene, benzyl ethyl ether, 1,1,2-tribromoethane and 5-ethyl-4-propyl-1,3-dioxane.

To prepare the salts of 2-[3-(aryl)isoxazol-5-yl]benzoic acid, the spiro compound is heated with a base having a $pK_A$ of 11 or more resulting in the abstraction of the hydrogen from the 4-position of the isoxazoline ring and the formation of a salt of 3-aryl-isoxazol-5-yl benzoic acid. Although a stoichiometric amount of base may be utilized, it is preferable to dissolve the base in water and utilize a slight excess. This reaction may be conducted at room temperature and atmospheric pressure. Temperatures and pressures above ambient may be utilized.

The specific base used is not critical. However, the use of strong bases increases the rate of reaction. Suitable bases include but are not limited to such bases as alkali metal hydroxides, alkaline earth hydroxides, alkali metal carbonates and aliphatic amines.

The use of a solvent in addition to the water present is desirable although not necessary to the practice of this aspect of the present invention. If a solvent is utilized, the specific solvent used is not critical. It is desirable, however, to utilize a water-miscible solvent in order to enhance the reaction of the spiro compound with the base. This is especially true if an aqueous base is a reactant. Useful solvents include water, alcohol, dioxane, tetrahydrofuran, dimethylsulfide and the like.

This invention also relates to the preparation of 2-(3-Aryl-5-isoxazol) benzoyl halides by treatment of 3'(Aryl)-spiro[isobenzofuran-1(3H),5'(4'H)-isoxazol]-3-one with and acid halide in the presence of water. More specifically, the benzoyl halides are prepared according to the following reaction:

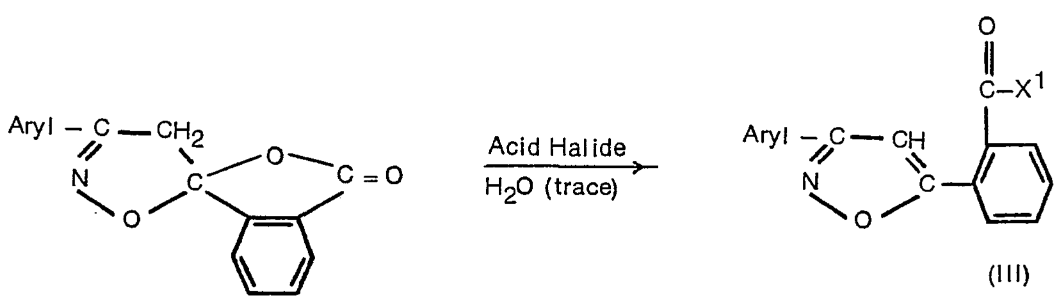


(III)

where $X^1$ is equal to Cl, Br, F or I and Aryl is as defined above.

Acid halides, especially acid chlorides, are well known in the art and include thionyl chloride, thionyl bromide, thionyl fluoride, phosphorus trichloride, phosphorus tribromide, oxalyl chloride, triphenyl phosphine-carbon tetrachloride, $PCl_5$, $POBr_3$ and the like. Specifically, reactive halides are those compounds containing halide ions that are known in the art to react with a carboxylic acid to form an acid halide.

To prepare the benzoyl halide, it is preferable to heat the spiro compound with the acid halide at

reflux. It is possible to utilize lower temperatures, as low as room temperature. However, their use will adversely effect the rate of reaction. The reaction readily proceeds at atmospheric pressure, although higher pressure could be utilized.

In accordance with the novel aspects of the present invention, it should be noted that the presence of a trace amount of water is required for reaction to proceed. If water were completely absent, the formation of the benzoyl halide would not occur. Typically, a few drops of water should be added to the reaction mixture. It is possible, however, that moisture in the atmosphere could provide sufficient water to drive the reaction. The critical factor is that a trace amount of water be provided from somewhere.

In order to illustrate the novel aspects of the present invention, the following examples are presented.

Example 1

Preparation of 2-[3-(m-Trifluoromethylphenyl)-5-Isoxazolyl]Benzoic Acid.

3′-(*m*-Trifluoromethylphenyl)spiro[isobenzofuran-(3H),5′(4′H)-isoxazol]-3-one (2 grams), m.p. 170°C was placed in a test tube and heated in an oil bath at 200—220°C. for two hours. NMR indicated that the reaction was complete and that the product was the desired acid. The crude product was a yellow solid. m.p. 173—175°C. Recrystallization from acetonitrile afforded 1.25 grams of a yellow solid; m.p. 177—178°C.

Alternatively, 2-[3-(*m*-trifluoromethylphenyl)isoxazol-5-yl] benzoic acid may be prepared according to the procedure of Example 2.

Example 2

Preparation of 2-[3-(m-Trifluoromethylphenyl)-5-Isoxazolyl]Benzoic Acid.

A solution of 3′-(*m*-trifluoromethylphenyl)spiro-[isobenzofuran-1(3H),5′(4′H)-isoxazol]-3-one (1 gram), m.p. 170°C, in 20 ml of *o*-dichlorobenzene was held at reflux for 3.5 hours, cooled and concentrated under vacuum to give a yellow solid. NMR indicated that the reaction was not yet complete. The solid was redissolved in 20 ml of *o*-dichlorobenzene and the solution was held at reflux for another 3 hours, cooled and concentrated under vacuum to give a quantitative yield of the desired acid as a pale yellow solid; m.p. 172—174°C.

Example 3

Preparation of 2-[3-(p-Trifluoromethylphenyl)-Isoxazol-5-yl]Benzoic Acid.

3′-(*p*-Trifluoromethylphenyl)-spiro[isobenzofuran-1(3H),5′(4′H)-isoxazol]-3-one (1.35 grams) m.p. 177—178°C. was placed in a test tube and heated in an oil bath at 200—220°C. for 35 minutes. NMR indicated that the reaction was complete and that the desired acid was prepared as a light brown solid; m.p. 198—202°C. Recrystallization from acetonitrile afforded 0.92 grams of a white-yellow solid; m.p. 205—207°C.

Anal. Calc'd for $C_{17}H_{10}F_3NO_3$. C, 61.27; H, 3.02.

Found: C, 61.22; H, 3.07.

Example 4

Preparation of 2-[3-(2,4-Dichlorophenyl)-Isoxazol-5-yl] Benzoic acid.

3′-(2,4-Dichlorophenyl)-spiro[isobenzofuran-1(3H),5′(4′H)-isoxazol]-3-one (1.35 grams) m.p. 158—161°C was placed in a test tube and heated with an oil bath at a temperature of 185—190°C. for about 15 minutes. NMR indicated the reaction was complete and that the desired acid was obtained as a white solid; m.p. 181—183°C. Recrystallization from acetonitrile resulted in 0.75 grams of a white solid; m.p. 182—183°C.

Anal. Calc'd for $C_{16}H_9NCl_2O_3$. C, 57.86; H, 2.73.

Found: C, 57.61; H, 2.79.

Example 5

Preparation of Sodium Salt of 2-[3-o-Methylphenyl)-5-Isoxazolyl]Benzoic Acid.

To 3′-(*o*-methylphenyl)-spiro[isobenzofuran-1(3H),5′(4′H)-isoxazol]-3-one (2.5 g, 0.0035 mole) m.p. 156—157°C in 25 ml of ethanol was added a solution 0.6 g (0.015 mole) of sodium hydroxide in 25 ml of water. The solution was stirred for two hours. An additional 50 ml of water was added and stirring continued resulting in a solution containing a salt of the desired acid. The solution was then acidified with concentrated hydrochloric acid and extracted with 200 ml of ether. The ethereal solution was washed two times with water, dried over calcium sulfate and concentrated under a with a vacuum to give 2.40 g of the desired acid as white crystals, m.p. 158.5—159.5°C. The crystals were recrystallized from 10 ml of acetonitrile to give 1.55 g of colorless crystals, m.p. 160.5—161.5°C.

Anal Calc'd: C, 73.11; H, 4.69.

Found: C, 73.08; H, 4.71.

Example 6

Preparation of Sodium Salt of 2-[3-m-Trifluoromethylphenyl)-5-isoxazolyl]Benzoic Acid.

To 1.0 g of 3'-(*m*-trifluoromethylphenyl)spiro[isobenzofuran-1(3H),5'(4'H)-isoxazol]-3-one, m.p. 170°C, dissolved in 25 ml of ethanol was added 20 ml of a 1 N sodium hydroxide solution. The solution was stirred at room temperature for five hours giving the sodium salt of the desired acid. The clear solution was acidified with concentrated hydrochloric acid, extracted with 300 ml of ether, washed two times with water, dried over calcium sulfate and concentrated to give 0.98 g of the desired acid as a white solid, m.p. 175—176°C.

Example 7

Preparation of Sodium Salt of 2-[3-m-Cyanophenyl)-5-Isoxazolyl]Benzoic Acid.

Utilizing the procedure of Example 1, and 3'-(3-cyanophenyl)-spiro[isobenzofuran-1(3H),5'(4'H)-isoxazol]-3-one, m.p. 165—167°C, as the starting material 2-[3-(m-cyanophenyl)-5-isoxazolyl] benzoic acid, m.p. 195—196°C., was prepared.

Anal. Calc'd: C, 70.34; H, 3.47.

Found: C, 70.22; H, 3.49.

Example 8

Preparation of 2-[3-trifluoromethylphenyl)-5-Isoxazol]Benzoyl Chloride.

A solution of 3'-(*m*-trifluoromethylphenyl)spiro[isobenzofuran-1(3H),5'(4'H)-isoxazol]-3-one (30 g, 0.90 mole), m.p. 170°C., thionyl chloride (150 ml) and water (5 drops) was held at reflux for 2 hours, cooled and concentrated under vacuum to 32.7 g of yellow solid, m.p. 86—89°C. Recrystallization of 10 g of the yellow solid from 50 ml. of carbon tetrachloride afforded 7.1 g of a pale yellow solid, m.p. 90.5—92°C. Recrystallization of 2.1 g of the pale yellow solid once again from carbon tetrachloride gave 1.47 g pure product as white crystals, m.p. 91—92°C.

Anal. Calc'd for $C_{17}H_9F_3ClNO_2$: C, 58.05; H, 2.58.

Found: C, 57.67; H, 2.57.

Example 9

Preparation of Isopropyl, 2-[3-(3-Trifluoromethylphenyl)Isoxazol-5-yl] Benzoic Acid Ester.

The benzoyl chloride of Example 8 was esterified as follows. 2-[3-[3-(trifluoromethyl) phenyl]-5-isoxazol] benzoyl chloride (5 g., 0.0162 mole) was added portionwise over 10 minutes to a stirred ice-cold solution of isopropanol (17 g., 0.202 mole) in 15 ml. of pyridine. The reaction mixture was allowed to stand at room temperature for one hour and then was taken up in 300 ml of ether and washed three times with 1 Normal HCl, one time with sodium carbonate and finally with $H_2O$. The ether solution was dried and concentrated to yield 4.94 g of the isopropyl ester as a yellow oil. The oil was chromatographed on a silica gel column with 50% ethyl acetate 50% hexane as eluant to give a total of 3.7 g of pure isopropyl ester, $n_D^{25} = 1.5426$.

Anal. Calc'd for $C_{20}H_{16}F_3NO_3$: C, 64.00; H, 4.30.

Found: C, 63.99; H, 4.31.

The above examples disclose an efficient process for preparing 3-aryl-isoxazol-5-yl benzoic acid, salts and halides thereof that are useful as herbicides and plant growth regulants. If desired, the acids may be esterified, as illustrated in Example 9, to give additional herbicides and plant growth regulants.

## Claims

1. A process for preparing 2-(3-Aryl-5-isoxazolyl)-benzoic acid having the formula

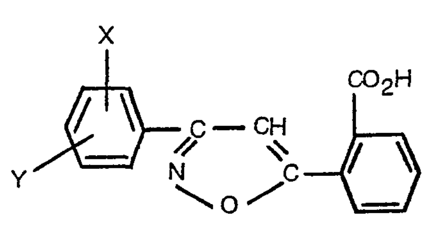

wherein X and Y are independently selected from the group consisting of hydrogen, halogen, $C_{1-5}$ alkyl, $C_{1-5}$ alkoxy, halo-$C_{1-5}$ alkyl, phenoxyphenyl and cyano, which comprises heating a molten 3'-(Aryl)spiro-[isobenzofuran-1(3H),5'(4'H)-isoxazol]-3-one having the formula

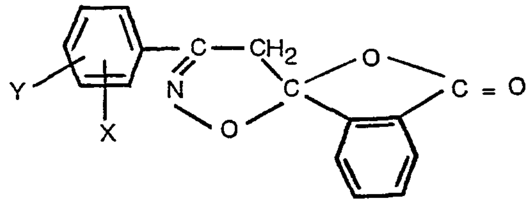

or a solution of a 3'-(Aryl)spiro[isobenzofuran-1(3H),5'(4'H)-isoxazol]-3-one having the said formula in an inert solvent at a temperature ranging from 100°C to the reflux temperature of the solution.

2. A process according to Claim 1 wherein said temperature is at least 125°C.

3. A process according to Claim 2 wherein a solution of the 3'-(Aryl)spiro[isobenzofuran-1(3H),5'(4'H)-isoxazol]-3-one in an inert solvent is heated at the reflux temperature of the solution.

4. A process according to Claim 1 wherein X is hydrogen and Y is trifluoromethyl.

5. A process for preparing a salt of 3-Aryl-isoxazol-5-yl benzoic acid which comprises reacting a spiro compound having the formula

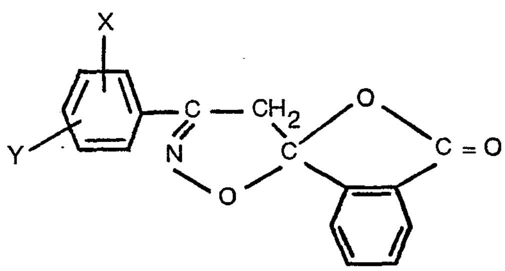

wherein X and Y are independently selected from the group consisting of hydrogen, halogen, $C_{1-5}$ alkyl, $C_{1-5}$ alkoxy, halo $C_{1-5}$ alkyl, phenoxy, phenyl and cyano, with a base having a $pK_A$ of 11 or more.

6. A process according to Claim 5 wherein said base is selected from the group consisting of alkali metal hydroxides, alkaline earth carbonates, alkali metal carbonates and aliphatic amines.

7. A process according to Claim 5 wherein the spiro compound is reacted with an aqueous solution of the base, optionally in the presence of a water-miscible solvent.

8. A process according to Claim 5 wherein X is hydrogen and Y is halo-$C_{1-5}$ alkyl.

9. A process according to Claim 8 wherein said spiro compound has the formula

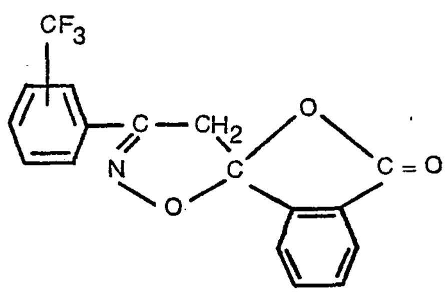

10. A process according to Claim 9 wherein said trifluoromethyl moiety is in the meta position.

11. A process according to Claim 9 wherein said base is an alkali metal hydroxide.

12. A process according to Claim 7 which comprises reacting a spiro compound having the formula

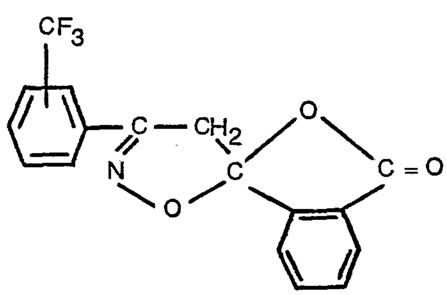

with sodium hydroxide in the presence of an alcohol.

13. A process according to Claim 7 further comprising the step of acidifying the resulting salt to form the free acid of said salt.

14. A process for preparing 2-(3-Aryl-5-isoxazol) benzoyl halide having the formula

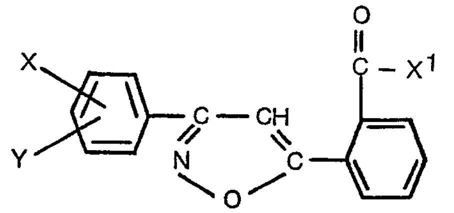

wherein X and Y are independently selected from the group consisting of hydrogen, halogen, $C_{1-5}$ alkyl, $C_{1-5}$ alkoxy, halo-$C_{1-5}$ alkyl, phenoxyphenyl and cyano and wherein $X^1$ is a chloro, bromo, fluoro or iodo; which comprises heating 3'-(Aryl)spiro[isobenzofuran-1(3H),5'(4'H)-isoxazol]-3-one having the formula

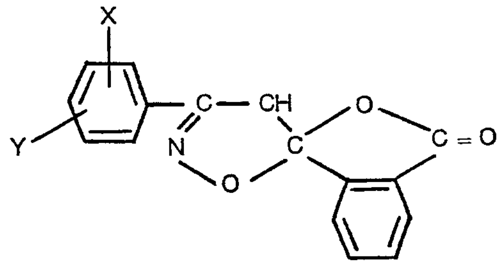

wherein X and Y are as previously defined, with an acid halide and water.

15. A process according to Claim 14 wherein X is hydrogen and Y is trifluoromethyl.

16. A process according to Claim 14 wherein said acid halide is selected from the group consisting of thionyl chloride, thionyl bromide, thionyl fluoride, phosphorus trichloride, phosphorus tribromide, oxalyl chloride, triphenyl phosphine-carbon tetrachloride, $PCl_5$, $POBr_3$ and $POCl_3$.

17. A process according to Claim 16 wherein said acid halide is selected from the group consisting of thionyl chloride, phosphorus trichloride, $PCl_5$ and $POCl_3$.

18. A process according to Claim 14 wherein X' is chlorine.

19. A process according to Claim 18 wherein said acid halide is thionyl chloride.

**Patentansprüche**

1. Verfahren zur Herstellung von 2-(3-Aryl-5-isoxazolyl)-benzoesäure der Formel

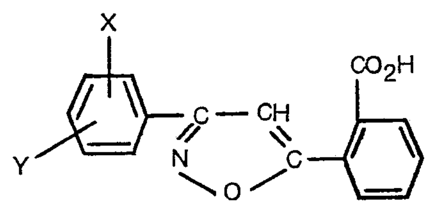

in der X und Y unabhängig aus der Gruppe von Wasserstoff, Halogen, $C_{1-5}$-Alkyl, $C_{1-5}$-Alkoxy, Halogen-$C_{1-5}$-alkyl, Phenoxyphenyl und Cyano ausgewählt sind, dadurch gekennzeichnet, daß ein geschmolzenes 3'-(Aryl)spiro[isobenzofuran-1(3H),5'(4'H)-isoxazol]-3-on der Formel

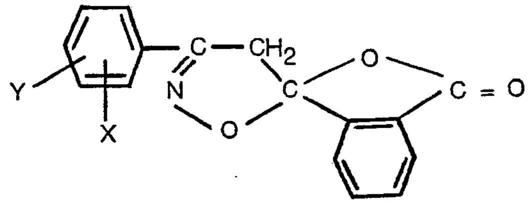

oder eine Lösung eines 3'-(Aryl)spiro[isobenzofuran-1(3H),5'(4'H)-isoxazol]-3-ons der angegebenen Formel in einem inerten Lösungsmittel bei einer Temperatur im Bereich von 100°C bis zur Rückflußtemperatur der Lösung erhitzt wird.

2. Verfahren nach Anspruch 1, dadurch gekennzeichnet, daß die Temperatur mindestens 125°C. beträgt.

3. Verfahren nach Anspruch 2, dadurch gekennzeichnet, daß eine Lösung von 3'-(Aryl)spiro[isobenzofuran-1(3H),5'(4'H)-isoxazol]-3-on in einem inerten Lösungsmittel auf die Rückflußtemperatur der Lösung erhitzt wird.

4. Verfahren nach Anspruch 1, dadurch gekennzeichnet, daß X ein Wasserstoffatom und Y eine Trifluormethylgruppe darstellt.

5. Verfahren zur Herstellung eines Salzes der 3-Aryl-isoxazol-5-yl-benzoesäure, dadurch gekennzeichnet, daß eine Spiro-Verbindung der Formel

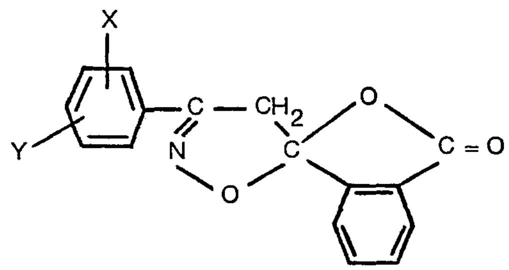

in der X und Y unabhängig aus der Gruppe von Wasserstoff, Halogen, $C_{1-5}$-Alkyl, $C_{1-5}$-Alkoxy, Halogen-$C_{1-5}$-alkyl, Phenoxy, Phenyl und Cyano ausgewählt sind, mit einer Base, die einen $pK_A$-Wert von mindestens 11 aufweist, umgesetzt wird.

6. Verfahren nach Anspruch 5, dadurch gekennzeichnet, daß die Base aus der Gruppe von Alkalimetallhydroxiden, Erdalkalimetallcarbonaten, Alkalimetallcarbonaten und aliphatischen Aminen ausgewählt ist.

7. Verfahren nach Anspruch 5, dadurch gekennzeichnet, daß die Spiro-Verbindung mit einer wäßrigen Lösung der Base, gegebenenfalls in Gegenwart eines mit Wasser mischbaren Lösungsmittels umgesetzt wird.

8. Verfahren nach Anspruch 5, dadurch gekennzeichnet, daß X ein Wasserstoffatom darstellt und Y eine Halogen-$C_{1-5}$-alkylgruppe darstellt.

9. Verfahren nach Anspruch 8, dadurch gekennzeichnet, daß die Spiro-Verbindung die Formel

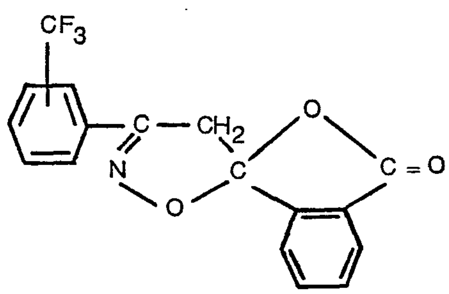

aufweist.

10. Verfahren nach Anspruch 9, dadurch gekennzeichnet, daß die Trifluormethylgruppe in meta-Position steht.

11. Verfahren nach Anspruch 9, dadurch gekennzeichnet, daß die Base ein Alkalimetallhydroxid ist.

12. Verfahren nach Anspruch 7, dadurch gekennzeichnet, daß eine Spiro-Verbindung der Formel

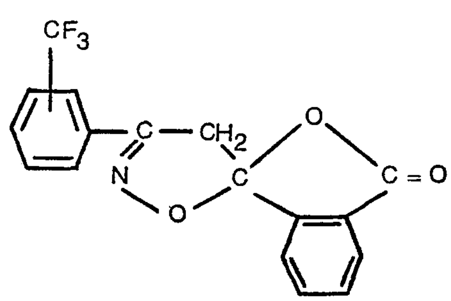

mit Natriumhydroxid in Gegenwart eines Alkohols umgesetzt wird.

13. Verfahren nach Anspruch 7, dadurch gekennzeichnet, daß in einem weiteren Schritt das erhaltene Salz acidifiziert wird, um die freie Säure des Salzes zu bilden.

14. Verfahren zur Herstellung von 2-(3-Aryl-5-isoxazol)-benzoylhalogenid der Formel

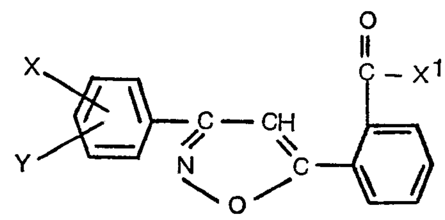

in der X und Y unabhängig aus der Gruppe von Wasserstoff, Halogen, $C_{1-5}$-Alkyl, $C_{1-5}$-Alkoxy, Halogen-$C_{1-5}$-alkyl, Phenoxyphenyl und Cyano ausgewählt sind und worin $X^1$ ein Chlor-, Brom-, Fluor- oder Jodatom darstellt, dadurch gekennzeichnet, daß ein 3′-(Aryl)spiro[isobenzofuran-1(3H),5′(4′H)-isoxazol]-3-on der Formel

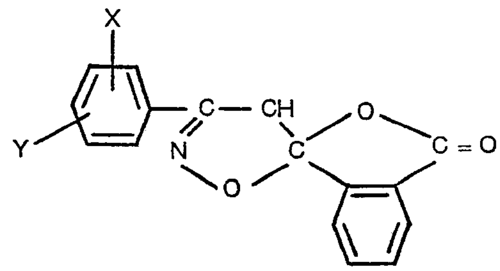

in der X und Y die vorstehend genannte Bedeutung haben, mit einem Säurehalogenid und Wasser erhitzt wird.

15. Verfahren nach Anspruch 14, dadurch gekennzeichnet, daß X ein Wasserstoffatom darstellt und Y eine Trifluormethylgruppe bedeutet.

16. Verfahren nach Anspruch 14, dadurch gekennzeichnet, daß das Säurehalogenid aus der Gruppe von Thionylchlorid, Thionylbromid, Thionylfluorid, Phosphortrichlorid, Phosphortribromid, Oxalylchlorid, Triphenylphosphin-Kohlenstofftetrachlorid, $PCl_5$, $POBr_3$ und $POCl_3$ ausgewählt ist.

17. Verfahren nach Anspruch 16, dadurch gekennzeichnet, daß das Säurehalogenid aus der Gruppe bestehend aus Thionylchlorid, Phosphortrichlorid, $PCl_5$ und $POCl_3$ ausgewählt ist.

18. Verfahren nach Anspruch 14, dadurch gekennzeichnet, daß X′ Chlor bedeutet.

19. Verfahren nach Anspruch 18, dadurch gekennzeichnet, daß das Säurehalogenid Thionylchlorid ist.

**Revendications**

1. Procédé de préparation d'acide 2-(3-aryl-5-isoxazolyl)-benzoïque ayant la formule:

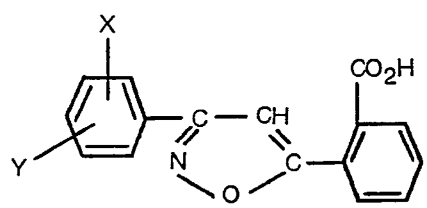

où X et Y sont indépendamment choisis dans le groupe se composant d'hydrogène, d'halogène, de groupes alkyle en $C_{1-5}$, alcoxy en $C_{1-5}$, halo-alkyle en $C_{1-5}$, phénoxyphényle et cyano, qui consiste à chauffer une 3′-(aryl)spiro[isobenzofuran-1(3H),5′(4′H)-isoxazol]-3-one en fusion ayant la formule:

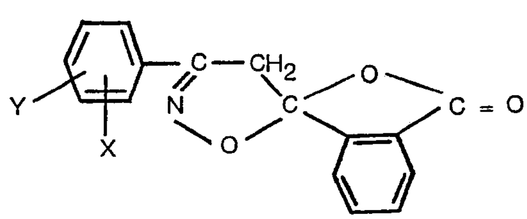

ou une solution d'une 3′-(aryl)spiro[isobenzofuran-1(3H),5′(4′H)-isoxazol]-3-one ayant la formule indiquée dans un solvant inerte, à une température allant de 100°C jusqu'à la température de reflux de la solution.

2. Procédé selon la revendication 1, dans lequel la température est au moins 125°C.

3. Procédé selon la revendication 2, dans lequel une solution de la 3′-(aryl)spiro[isobenzofuran-1(3H),5'(4'H)-isoxazol]-3-one dans un solvant inerte est chauffée à la température du reflux de la solution.

4. Procédé selon la revendication 1, dans lequel X est l'hydrogène et Y est le groupe trifluorométhyle.

5. Procédé de préparation d'un sel d'acide 3-aryl-isoxazol-5-yl benzoïque qui consiste à faire réagir un composé spiro ayant la formule:

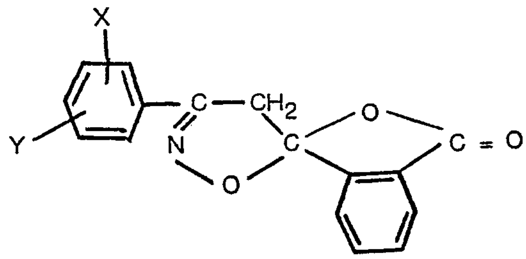

où X et Y sont indépendamment choisis dans le groupe se composant d'hydrogène, d'halogène, de groupes alkyle en $C_{1-5}$, alcoxy en $C_{1-5}$, halo-alkyle en $C_{1-5}$, phénoxy, phényle et cyano, avec une base ayant un $pK_A$ de 11 ou davantage.

6. Procédé selon la revendication 5, dans lequel la base est choisie dans le groupe se composant d'hydroxydes de métaux alcalins, de carbonates de métaux alcalino-terreux, de carbonates de métaux alcalins et d'amines aliphatiques.

7. Procédé selon la revendication 5, dans lequel le composé spiro est mis à réagir avec une solution aqueuse de la base, de manière facultative en présence d'un solvant miscible avec l'eau.

8. Procédé selon la revendication 5, dans lequel X est l'hydrogène et Y est un groupe halo-alkyle en $C_{1-5}$.

9. Procédé selon la revendication 8, dans lequel le composé spiro a la formule:

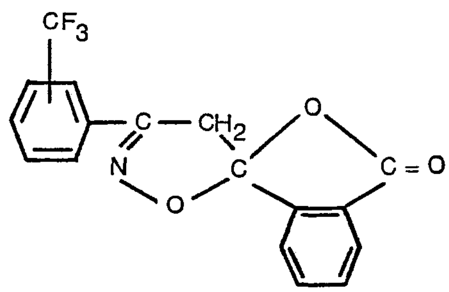

10. Procédé selon la revendication 9, dans lequel la partie trifluorométhyle est en position metá.

11. Procédé selon la revendication 9, dans lequel la base est un hydroxyde de métal alcalin.

12. Procédé selon la revendication 7, qui consiste à faire réagir un composé spiro ayant la formule:

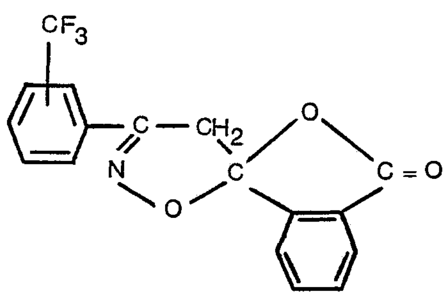

avec la soude, en présence d'un alcool.

13. Procédé selon la revendication 7, comprenant en outre l'étape d'acidification du sel résultant pour former l'acide libre de ce sel.

14. Procédé de préparation d'halogénure de 2-(3-aryl-5-isoxazol)benzoyle ayant la formule:

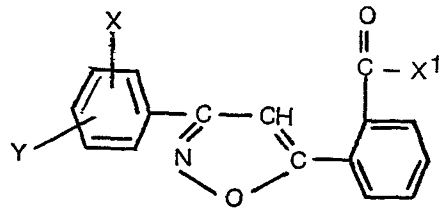

où X et Y sont indépendamment choisis dans le groupe se composant d'hydrogène, d'halogène, de groupes alkyle en $C_{1-5}$, alcoxy en $C_{1-5}$, halo-alkyle en $C_{1-5}$, phénoxyphényle et cyano et où $X^1$ est un atome de chlore, de brome, de fluor, ou d'iode, qui consiste à chauffer une 3'-(aryl)spiro[isobenzofuran-1(3H),5'(4'H)-isoxazol]-3-one ayant la formule:

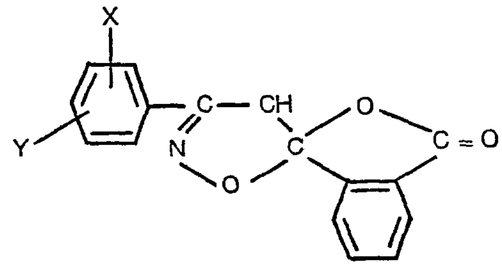

où X et Y sont comme définis préalablement, avec un halogénure d'acide et de l'eau.

15. Procédé selon la revendication 14, dans lequel X est l'hydrogène et Y est le groupe trifluorométhyle.

16. Procédé selon la revendication 14, dans lequel l'halogénure d'acide est choisi dans le groupe se composant de chlorure de thionyle, de bromure de thionyle, de fluorure de thionyle, de trichlorure de phosphore, de tribromure de phosphore, de chlorure d'oxalyle, de triphénylphosphine-tétrachlorure de carbone, de $PCl_5$, de $POBr_3$ et de $POCl_3$.

17. Procédé selon la revendication 16, dans lequel l'halogénure d'acide est choisi dans le groupe se composant de chlorure de thionyle, de trichlorure de phosphore, de $PCl_5$ et de $POCl_3$.

18. Procédé selon la revendication 14, dans lequel $X^1$ est le chlore.

19. Procédé selon la revendication 18, dans lequel l'halogénure d'acide est le chlorure de thionyle.